(19) Europäisches Patentamt
European Patent Office
Office européen des brevets

(11) Veröffentlichungsnummer: **0 023 612**
**B1**

(12) **EUROPÄISCHE PATENTSCHRIFT**

(45) Veröffentlichungstag der Patentschrift: **15.06.83**

(51) Int. Cl.³: **C 07 D 321/00**, C 07 C 69/34, C 07 C 67/08, C 08 G 63/16, C 11 B 9/00, A 61 K 7/46

(21) Anmeldenummer: **80104034.6**

(22) Anmeldetag: **12.07.80**

# (54) Makrocyclische Diester, Verfahren zu ihrer Herstellung und ihre Verwendung als Riechstoffe.

(30) Priorität: **24.07.79 DE 2929864**

(43) Veröffentlichungstag der Anmeldung: **11.02.81 Patentblatt 81/6**

(45) Bekanntmachung des Hinweises auf die Patenterteilung: **15.06.83 Patentblatt 83/24**

(84) Benannte Vertragsstaaten: **CH DE FR GB IT LI NL**

(56) Entgegenhaltungen:

**CH-A-589 642**
**DE-A-1 929 550**
**DE-B-2 547 267**

**CHEMISCHES ZENTRALBLATT, Band 98, Nr. 20, 1927 II, Berlin L. RUZICKA et al. »Gewinnung einiger mit der Untersuchung des Muscons zusammenhängender Methylpolymethylendicarbonsäuren. I. Synthesen ausgehend von Citronellal« Seiten 2450 bis 2451**
**Chemical Abstracts Band 43, Nr. 1, 1949 Columbus, Ohio, USA M. STOLL et al. »Syntheses of macrocyclic products of a musk odor. XII. Reduction of beta-methylcyclopentadecanoin to muscone and 4-methylcyclopentadecanone« Spalte 163h**
**Patents of Abstracts of Japan Band 2, Nr. 122, 13. Oktober 1978 Seite 2375C78**
**Patents Abstracts of Japan Band 4, Nr. 26, 6. März 1980 Seite 102C1**
**Patents Abstracts of Japan Band 3, Nr. 138, 16. November 1979 Seite 27C64**

(73) Patentinhaber: **HAARMANN & REIMER GMBH, Postfach 138, D-3450 Holzminden (DE)**

(72) Erfinder: **Bauer, Kurt, Dr., Corveyblick 41, D-3450 Holzminden (DE)**
Erfinder: **Körber, Alfred, Dr., Bismarckstrasse 4, D-3450 Holzminden (DE)**
Erfinder: **Bork, Karl-Heinz, Bergblick 17, D-3450 Holzminden (DE)**

(74) Vertreter: **Dill, Erwin, Dr. et al, c/o BAYER AG Zentralbereich Patente Marken und Lizenzen Bayerwerk, D-5090 Leverkusen 1 (DE)**

**Patents Abstracts of Japan Band 3, Nr. 123, 16. Oktober 1979 Seite 133C61**
**Chemical Abstracts Band 78, Nr. 25, 1973 Columbus, Ohio, USA T. YASUKAWA et al. »Large ring ethylenedioates« Seite 426, Spalte 1, Abstract Nr. 159697g**
**Chemical Abstracts Band 82, Nr. 24, 1975 Columbus, Ohio, USA T. KUWATA et al. »Perfume containing ethylene dodecanedioate« Seite 261, Spalte 1, Abstract Nr. 160115n**
**Chemical Abstracts Band 89, Nr. 23, 1978 Columbus, Ohio, USA N. KAWABE et al. »Macrocyclic ethylenedioates« Seite 640, Spalte 2, Abstract Nr. 197620u**
**Chemical Abstracts Band 79, Nr. 21, 1973 Columbus, Ohio, USA K. SCHABACHER et al. »Lipomycins. II. Structure of alpha- and betalipomycin« Seite 433, Spalte 1, Abstract Nr. 126740r**

Die Akte enthält technische Angaben, die nach dem Eingang der Anmeldung eingereicht wurden und die nicht in dieser Patentschrift enthalten sind.

EP 0 023 612 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Makrocyclische Diester, Verfahren zu ihrer Herstellung und ihre Verwendung als Riechstoffe

Die Erfindung betrifft makrocyclische Diester der Formel

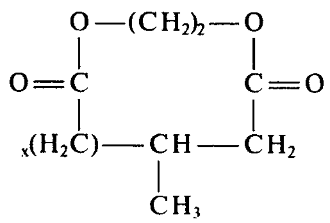

(I)

in der x für eine ganze Zahl von 8 bis 11 steht.

Die Erfindung betrifft ferner ein Verfahren zur Herstellung der Diester der Formel (I). Das Verfahren ist dadurch gekennzeichnet, daß man eine Verbindung der allgemeinen Formel

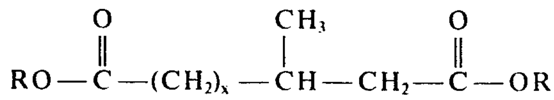

(II)

in der

R für ein Wasserstoffatom oder einen $C_1-C_4$-Alkylrest und
x für eine ganze Zahl von 8 bis 11 steht,

mit Ethylenglykol zu einem Polyester umsetzt und diesen anschließend depolymerisiert.

Außerdem betrifft die Erfindung die Verwendung der makrocyclischen Diester der Formel (I) als Riechstoffe.

Die Herstellung des Polyesters kann durch Umsetzung von Verbindungen der allgemeinen Formel (II), in der R eine $C_1-C_4$-Alkylgruppe, vorzugsweise eine Methyl- oder Ethylgruppe bedeutet, mit Ethylenglykol in Gegenwart von Säuren oder Basen erfolgen.

Als Säuren eignen sich starke Säuren, wie Schwefelsäure, wasserfreier Chlorwasserstoff, Toluolsulfonsäure oder stark saure Kationenaustauscher. Als Basen kommen Alkalihydroxide und -alkoholate wie Natriumhydroxid, Kaliumhydroxid, Lithiumhydroxid, Natriummethylat, Natriumethylat oder vorzugsweise metallisches Natrium in Frage. Säuren und Basen werden in für Umesterungskatalysatoren üblichen Mengen eingesetzt.

Die Herstellung des Polyesters kann auch durch Veresterung der Disäuren der allgemeinen Formel (II) mit Ethylenglykol in Gegenwart einer der obengenannten starken Säuren erfolgen. Dabei wird der Alkohol bevorzugt im Überschuß eingesetzt und das sich bildende Reaktionswasser mit einem Schleppmittel wie Chloroform oder Tetrachlorkohlenstoff aus dem Reaktionsgemisch entfernt.

Vorzugsweise erfolgt die Herstellung des Polyesters aber durch Veresterung der Disäuren der Formel (II) mit Ethylenglykol ohne Katalysator und ohne Schleppmittel bei Temperaturen von 120 bis 200°C, vorzugsweise 160 bis 180°C.

Der Polyester wird thermisch, z. B. durch Erhitzen auf 250 bis 300°C, depolymerisiert. Die Depolymerisierung kann gegebenenfalls in Gegenwart üblicher Depolymerisierungskatalysatoren, wie Magnesiumchlorid, Magnesiumoxid oder Bleidioxid, vorgenommen werden.

Die Verbindungen der allgemeinen Formel (II) können wie folgt hergestellt werden: Korksäure, Azelainsäure, Sebacinsäure oder Nonan dicarbonsäure werden nach bekannten Verfahren in die Halbesterchloride überführt. Diese Halbesterchloride werden mit Dimethylacrylsäureethylester in Gegenwart eines Friedel-Crafts-Katalysators zu einfach ungesättigten 3-Methyl-5-oxo-disäurediethylestern umgesetzt. Diese Ester werden durch Hydrieren in Gegenwart von Raney-Nickel und nachfolgende Wolff-Kishner-Reduktion mit Hydrazin in die Disäuren der allgemeinen Formel (II) überführt. Die so erhaltenen Disäuren können nach bekannten Veresterungsverfahren in die Diester der Formel (II) überführt werden.

Die erfindungsgemäßen makrocyclischen Diester sind wertvolle Riechstoffe. Sie weisen gegenüber dem bekannten Brassylsäureethylenester (siehe z. B. US-A-4 165 321) überraschenderweise den Vorteil auf, daß sie neben einer Moschusnote deutliche erogene Akzente aufweisen, die an Costuswurzelöl erinnern. Sie können u. a. daher als Ausgangsbasis für neuartige, erogene, langhaftende Duftnoten dienen oder in Kombination mit anderen Moschuskörpern eingesetzt werden, um diese zu verstärken und natürlicher zu gestalten.

Die erfindungsgemäßen Verbindungen werden in Mischungen mit anderen Riechstoffen in Riechstoffkompositionen, z. B. in Mengen von 0,01 bis 25 Gewichtsprozent, bezogen auf das Gesamtgewicht eingesetzt.

Das Anwendungsgebiet der erfindungsgemäßen Verbindungen ist wegen ihres harmonischen

Geruchstyps und ihrer vorteilhaften anwendungstechnischen Eigenschaften, z. B. der Stabilität gegenüber aggressiven Medien, ungewöhnlich groß. Sie eignen sich für die Verwendung in Parfümkompositionen für die verschiedensten Fertigungsprodukte z. B. für hochwertige Kosmetika, für Feinparfümerieartikel wie Extraits, Seifen, Deo-Sprays, Shampoos, Schaumbäder und für Detergentien.

## Beispiel 1

366 g (1,5 Mol) 3-Methyl-dodecandisäure werden mit 186 g (3 Mol) Ethylenglykol unter Rühren auf 170°C erhitzt. Dabei werden 20 g (1,1 Mol) Wasser abgespalten. Der entstandene Polyester wird im Vakuum (2,7 mbar) von restlichem Wasser und überschüssigem Ethylenglykol befreit und danach mit 7,5 g Magnesiumoxid vermischt. Dieses Gemisch wird in eine Destillationsapparatur getropft, deren Blase auf 290°C erhitzt wird und die unter einem Vakuum von 0,9 bis 1,5 mbar gehalten wird. Bei 127 bis 135°C/1,2 mbar destillieren 324 g Rohprodukt über. Daraus werden durch Feindestillation 311 g 7-Methyl-1,4-dioxa-cyclohexadecan-5,16-dion mit einem Siedepunkt von 125 bis 128°C/0,4 mbar erhalten, entsprechend einer Ausbeute von 76,8% der Theorie, bezogen auf eingesetzte Disäure.

Die als Ausgangsmaterial verwendete 3-Methyl-dodecandisäure wird nach folgendem Verfahren hergestellt:

499,2 g (2,4 Mol) Korksäure-ethylester-chlorid und 307 g (2,4 Mol) Dimethylacrylsäureethylester werden gleichzeitig innerhalb von 7 Stunden zu einer Suspension von 957,6 g (7,3 Mol) Aluminiumchlorid in 750 ml Methylenchlorid dosiert, wobei die Temperatur durch Kühlen auf 20 bis 27°C gehalten wird. Nach beendeter Zugabe wird die Reaktionsmischung zunächst 3 Stunden auf Rückflußtemperatur erhitzt, dann mit Wasser/Eis hydrolysiert.

Die wäßrige Phase wird zweimal mit Methylenchlorid extrahiert. Die vereinigten organischen Phasen werden neutralgewaschen und das Lösungsmittel abgezogen. Man erhält 748 g rohen 3-Methyl-5-oxo-dodecen-2(3)-disäurediethylester, der ohne weitere Reinigung mit 295,1 g (5,27 Mol) Kaliumhydroxid, 1944 ml Ethanol und 1171 ml Wasser in einen Autoklaven gegeben und unter Zusatz von 100 g Raney-Nickel bei 40°C und 40,5 bar Wasserstoffdruck hydriert wird. Die Wasserstoffaufnahme ist nach 3,5 Stunden beendet. Nach Abfiltrieren des Katalysators wird die Reaktionslösung mit konzentrierter Salzsäure angesäuert. Zunächst wird am Dünnschichtverdampfer die Hauptmenge des Ethanols aus der Reaktionslösung abgetrennt. Der Rückstand wird in Ether aufgenommen und neutralgewaschen. Nach dem Abziehen des Lösungsmittels verbleiben 588 g rohe 3-Methyl-5-oxo-dodecandisäure.

Die 588 g rohe 3-Methyl-5-oxo-dodecandisäure werden ohne weitere Reinigung mit 638,4 g (11,4 Mol) Kaliumhydroxid, 2,4 l Diethylenglykol und 427 g (6,84 Mol) 80%igem Hydrazinhydrat 2 Stunden auf Rückflußtemperatur erhitzt. Dann werden Wasser und überschüssiges Hydrazin abdestilliert. Die Reaktionstemperatur wird so lange auf 190 bis 200°C gehalten, bis keine Stickstoffentwicklung mehr stattfindet. Nach dem Abkühlen wird das Reaktionsprodukt auf Eis gegossen, mit Salzsäure angesäuert und mit Essigester extrahiert. Nach dem Abziehen des Lösungsmittels verbleiben 366 g 3-Methyl-dodecandisäure (Fp: 81—82°C), entsprechend einer Ausbeute von 62,5% der Theorie, bezogen auf das Korksäurehalbesterchlorid.

## Beispiel 2

198 g (0,77 Mol) 3-Methyl-tridecandisäure werden unter den in Beispiel 1 für die Veresterung von 3-Methyl-dodecandisäure beschriebenen Bedingungen mit 95 g (1,54 Mol) Ethylenglykol zum Polyester kondensiert. Der Polyester wird anschließend ebenfalls unter den in Beispiel 1 beschriebenen Bedingungen in Gegenwart von 4 g Magnesiumoxid depolymerisiert. Man erhält 170,6 g Rohprodukt und nach einer Feindestillation 160,3 g 7-Methyl-1,4-dioxa-cycloheptadecan-5,17-dion mit einem Siedepunkt von 140°C/0,53 mbar, entsprechend einer Ausbeute von 73,3% der Theorie, bezogen auf eingesetzte Disäure.

Das Ausgangsmaterial 3-Methyl-tridecandisäure (Fp: 68,5—69,5°C) wird unter den in Beispiel 1 für die Herstellung von 3-Methyl-dodecandisäure beschriebenen Bedingungen aus Azelainsäureethylesterchlorid und Dimethylacrylsäureethylester hergestellt.

## Beispiel 3

101 g (0,31 Mol) 3-Methyl-tetradecandisäurediethylester und 39 g (0,64 Mol) Ethylenglykol werden auf 110°C erhitzt. Nach Zugabe von 0,5 Natrium wird die Temperatur auf 180°C gesteigert. Innerhalb von 2 Stunden destillieren 23,4 g Ethanol über. Durch Anlegen von Vakuum werden restliches Ethanol und überschüssiges Ethylenglykol abdestilliert. Der Rückstand wird in Chloroform aufgenommen und mit Salzsäure angesäuert. Die organische Phase wird neutralgewaschen und das Lösungsmittel

abgezogen. Es werden 87,7 g roher Polyester erhalten, die ohne weitere Reinigung mit 1,7 g Magnesiumoxid vermischt und wie in Beispiel 1 beschrieben depolymerisiert werden. Bei 140 bis 160° C/1,3 mbar geht ein Rohprodukt über, das nach Feindestillation 74 g 7-Methyl-1,4-dioxa-cyclooctadecan-5,18-dion mit einem Siedepunkt von 143° C/0,4 mbar liefert, entsprechend einer Ausbeute von 79% der Theorie, bezogen auf eingesetzten Disäurediethylester.

Der als Ausgangsmaterial verwendete 3-Methyl-tetradecandisäurediethylester wird wie folgt hergestellt:
Aus 782 g (3,15 Mol) Sebacinsäureethylesterchlorid und 403 g (3,15 Mol) Dimethylacrylsäureethylester werden nach der in Beispiel 1 für die Herstellung von 3-Methyl-dodecansäure beschriebenen Arbeitsweise durch Kondensation, Hydrieren und Wolff-Kishner-Reduktion 538 g (1,98 Mol) 3-Methyl-tetradecandisäure vom Schmelzpunkt 75° C erhalten.

Diese 538 g (1,98 Mol) 3-Methyl-tetradecandisäure werden mit 631 ml Ethanol und 74,2 g konzentrierter Schwefelsäure 4 Stunden auf Rückflußtemperatur erhitzt. Dann wird überschüssiges Ethanol abdestilliert, der Rückstand auf Wasser gegeben und mit Toluol extrahiert. Nach fraktionierter Destillation erhält man 616 g 3-Methyl-tetradecandisäureethylester mit einem Siedepunkt von 175 bis 185° C/0,8 mbar, entsprechend einer Ausbeute von 59,6% der Theorie, bezogen auf eingesetztes Sebacinsäureethylesterchlorid.

Beispiel 4

Eine Chyprekomposition wird durch Mischen folgender Komponenten hergestellt:

150 Bergamottöl Reggio
30 Citronenöl italienisch
50 Linalool
50 Benzylacetat
30 Methyldihydrojasmonat
30 Jasmin absolue marokkanisch
3 Dihydrojasmon
100 α-Hexylzimtaldehyd
38 Phenylethylalkohol
30 Citronellol
20 Eugenol
1 Cuminöl
10 Allylionon
5 Undecalacton
80 γ-Iraldein
50 Vetiverylacetat
20 Sandelholzöl ostindisch
30 Patschuliöl
50 Eichenmoos absolue jugoslawisch
3 Vanillin
780 Gewichtsteile

Durch Zusatz von 15 Gewichtsteilen 7-Methyl-1,4-dioxacycloheptadecan-5,17-dion erhält die Base bereits im Angeruch mehr Strahlung und einen höheren Impact. Im Nachgeruch wird die Base um eine interessante erogene Note bereichert, die nicht aufdringlich wirkt, sondern der Komposition eine weiche, weibliche Note verleiht.

Ähnliche Ergebnisse werden bei der Zugabe von 10 Gewichtsteilen 7-Methyl-1,4-dioxa-cyclohexadecan-5,15-dion bzw. 8 Gewichtsteilen 7-Methyl-1,4-dioxa-cyclooctadecan-5,18-dion erhalten.

**Patentansprüche**

1. Makrocyclische Diester der allgemeinen Formel

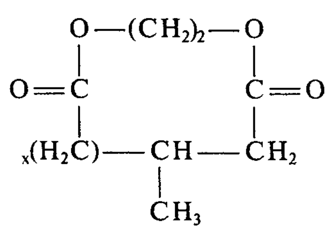

in der x für eine ganze Zahl von 8 bis 11 steht.

2. Verfahren zur Herstellung von makrocyclischen Diestern nach Anspruch 1, dadurch gekennzeichnet, daß man eine Verbindung der allgemeinen Formel

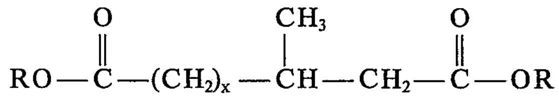

in der

R für Wasserstoffatom oder einen $C_1-C_4$-Alkylrest und
x für eine ganze Zahl von 8 bis 11 steht,

mit Ethylenglykol zu einem Polyester umsetzt und diesen anschließend depolymerisiert.

3. Verwendung der makrocyclischen Diester nach Anspruch 1 als Riechstoffe.

**Claims**

1. Macrocyclic diesters of the general formula

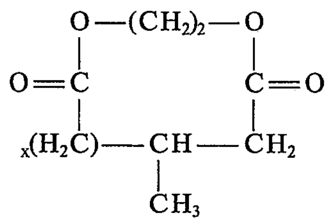

in which x represents an integer from 8 to 11.

2. Process for the preparation of macrocyclic diesters according to Claim 1, characterised in that a compound of the general formula

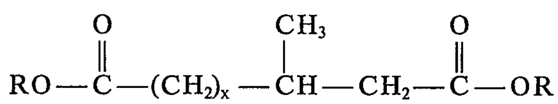

in which

R represents a hydrogen atom or a $C_1-C_4$ alkyl radical and
x represents an integer from 8 to 11,

is reacted with ethylene glycol to give a polyester which is then depolymerised.

3. Use of the macrocyclic diesters according to Claim 1 as odorants.

**Revendications**

1. Diester macrocyclique de formule générale

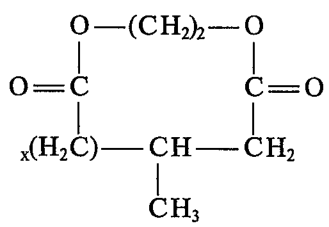

dans laquelle x représente un nombre entier valant 8 à 11.

2. Procédé de production de diesters macrocycliques selon la revendication 1, caractérisé en ce qu'on fait réagir un composé de formule générale

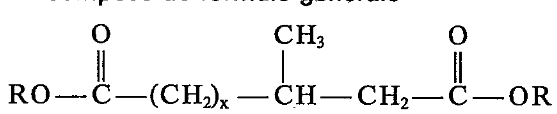

dans laquelle

R représente un atome d'hydrogène ou un radical alkyle ayant 1 à 4 atomes de carbone, et
x est un nombre entier valant 8 à 11,

avec l'éthylèneglycol pour obtenir un polyester que l'on dépolymérise ensuite.

3. Application des diesters macrocycliques selon la revendication 1 comme parfums.